# EUROPÄISCHE PATENTANMELDUNG

(11) **EP 4 205 723 A1**
(43) Veröffentlichungstag der Anmeldung: **05.07.2023**
(21) Anmeldenummer: 21218209.1
(22) Anmeldetag: 29.12.2021
(51) Int. Cl.: A61K 6/30, A61K 6/61, A61K 6/62, A61K 6/73, A61K 6/74, A61K 6/77, A61K 6/887, A61K 6/889

(54) **SULFATHALTIGER ODER PHOSPHATALTIGER, SELBSTHAFTENDER DENTALER KOMPOSITZEMENT MIT GUTER TRANSPARENZ**

(71) Anmelder: Ivoclar Vivadent AG, 9494 Schaan (LI)
(72) Erfinder: MOSZNER, Norbert, 9495 Triesen (LI); CATEL, Yohann, 9475 Rans (Sevelen) (CH); GIANASMIDIS, Alexandros, 9436 Balgach (CH); CATEL, Delphine, 9475 Rans (Sevelen) (CH); BOCK, Thorsten, 6800 Feldkirch (AT); GRABENBAUER, Barbara, 9445 Rebstein (CH); BROT, Andy, 9496 Balzers (LI); HALDNER, Tim, 9493 Mauren (LI)
(74) Vertreter: Uexküll & Stolberg

(57) **Zusammenfassung**

Radikalisch polymerisierbare Zusammensetzung, die mindestens ein saures, radikalisch polymerisierbares Monomer, mindestens einen Fluoroaluminiumsilikatglasfüllstoff und/oder röntgenopaken Glasfüllstoff und mindestens ein wasserlösliches Sulfat und/oder Phosphat enthält.

## Beschreibung

Die vorliegende Erfindung betrifft lagerstabile, radikalisch polymerisierbare, selbsthaftende Komposite mit verbesserter Transparenz, die sich insbesondere als Dentalwerkstoffe eignen, z.B. als dentale Zemente, Füllungskomposite oder Verblendmaterialen sowie zur Herstellung von Inlays, Onlays oder Kronen.

Komposite werden im Dentalbereich hauptsächlich zur Herstellung von direkten und indirekten Füllungen, d.h. als direkte und indirekte Füllungskomposite, sowie als Zemente eingesetzt. Die polymerisierbare organische Matrix der Komposite besteht meistens aus einer Mischung von Monomeren, Initiatorkomponenten und Stabilisatoren. Als Monomere werden gewöhnlich Mischungen von Dimethacrylaten eingesetzt, die auch monofunktionelle und funktionalisierte Monomere enthalten können. Häufig eingesetzte Dimethacrylate sind 2,2-Bis[4-(2-hydroxy-3-methacryloyloxy-propyl)phenyl]propan (Bis-GMA), 1,6-Bis-[2-methacryloyloxyethoxycarbonylamino]-2,2,4-trimethylhexan (UDMA), die eine hohe Viskosität haben und Polymerisate mit guten mechanischen Eigenschaften und geringem Polymerisationsschrumpf ergeben. Als reaktive Verdünner finden vor allem Triethylenglycoldimethacrylat (TEGDMA), 1,10-Decandioldimethacrylat (D₃MA) oder Bis(3-methacryloyloxymethyl)-tricyclo-[5.2.1.0^{2,6}]decan (DCP) Anwendung. Monofunktionelle Methacrylate, wie z.B. p-Cumylphenoxyethylenglycolmethacrylat (CMP-1E), eignen sich ebenfalls zur Reduzierung der Viskosität und bewirken darüber hinaus eine Verringerung der Netzwerkdichte und einen erhöhten Doppelbindungsumsatz.

Zur Herstellung selbsthaftender Komposite werden stark saure Haftmonomere eingesetzt, wie z.B. 10-Methacryloyloxydecyldihydrogenphosphat (MDP), das die Zahnhartsubstanz ätzt und durch Ionenbeziehung eine Haftung auf Schmelz/Dentin bewirkt. Haftmonomere verleihen Kompositen selbsthaftende Eigenschaften und ermöglichen damit den Einsatz der Komposite ohne eine Vorbehandlung der Zahnhartsubstanz mit einem Schmelz-Dentin-Adhäsiv, was deren Verwendung besonders attraktiv macht.

Neben der organischen Matrix enthalten Komposite einen oder mehrere Füllstoffe, die meist mit einem polymerisationsfähigen Haftvermittler, wie z.B. 3-Methacryloyloxypropyltrimethoxysilan, oberflächenmodifiziert sind. Füllstoffe verbessern die mechanischen Eigenschaften (Festigkeit, Elastizitätsmodul, Abrasionsfestigkeit) und die Verarbeitungseigenschaften (Pastenkonsistenz, Stopfbarkeit) der Werkstoffe und verleihen ihnen Röntgenopazität.

Problematisch ist, dass saure Haftmonomere mit Füllstoffen häufig nachteilige Wechselwirkungen eingehen. Beispielsweise werden die sauren Haftmonomere durch die Bildung unlöslicher Salze an die Oberfläche der Füllstoffe gebunden, oder sie bilden bei der Lagerung mit aus den Füllstoffen herausgelösten Ionen schwerlösliche Salze. Dies führt zu einer deutlichen Verringerung der Haftmonomerkonzentration in der Harzmatrix, was mit einer Verminderung oder sogar einem Verlust der Hafteigenschaften verbunden ist. Komposite mit sauren Haftmonomeren haben daher nur eine begrenzte Lagerstabilität.

Die Aushärtung methacrylatbasierender Dentalmaterialien erfolgt durch radikalische Polymerisation, wobei je nach Anwendungsgebiet radikalische Photoinitiatoren, thermische Initiatoren oder Redox-Initiatorsysteme eingesetzt werden. Dualhärtende Systeme enthalten eine Kombination von Photoinitiatoren und Redoxinitiatoren.

Kompositzemente enthalten in der Regel Redoxsysteme, weil diese auch dann eine ausreichende Härtung gewährleisten, wenn eine Lichthärtung wegen einer unzureichenden Durchstrahlung nicht möglich ist. Meist werden Redoxinitiatorsysteme eingesetzt, die auf einer Mischung von Dibenzoylperoxid (DBPO) mit tertiären aromatischen Aminen, wie z.B. N,N-Diethanol-p-toluidin (DEPT), N,N-Dimethyl-sym.-xylidin (DMSX) oder N,N-Diethyl-3,5-di-tert.-butylanilin (DABA) basieren. Da die Radikalbildung bei DBPO/Amin-basierenden Redoxinitiatorsystemen durch starke Säuren und damit auch durch stark saure Haftmonomere sehr beeinträchtigt wird, werden bevorzugt Cumolhydroperoxid-haltige Redoxinitiatorsysteme in Kombination mit Thioharnstoffen, wie z.B. Acetylthioharnstoff, eingesetzt.

Um eine ausreichende Lagerstabilität der Redoxinitiatoren zu gewährleisten, werden Redox-Initiatorsystem-basierende Materialien meist als sog. 2-Komponenten-Systeme (2K) eingesetzt, wobei das Oxydationsmittel (Peroxid- oder Hydroperoxid) und das Reduktionsmittel (Amine, Sulfinsäuren, Barbiturate, Thioharnstoffe etc.) in voneinander getrennte Komponenten eingearbeitet werden. Diese werden erst kurz vor der Anwendung miteinander gemischt. Zum Mischen werden zunehmend Doppelschubspritzen verwendet, die getrennte zylindrische Kammern zur Aufnahme der Komponenten aufweisen. Die Komponenten werden mit zwei miteinander verbundenen Kolben gleichzeitig aus den Kammern herausgedrückt und in einer Düse miteinander gemischt. Um möglichst homogene Mischungen zu erhalten, ist es vorteilhaft, die Komponenten in etwa gleich großen Volumenanteilen miteinander zu mischen.

Herkömmliche Befestigungszemente, wie z.B. ZnO-Eugenol-Zemente, Zinkphosphat-Zemente, Glasionomer-Zemente (GIZ) und harzmodifizierte Glasionomer-Zemente (Resin Modified Glass lonomer Cements: RMGI) eigenen sich nicht zur Anwendung mit Doppelschubspritzen, weil sie eine Pulverkomponente enthalten, was ein Mischen der Komponenten erheblich erschwert. Außerdem haben Glasionomerzemente nur eine geringe Transparenz und relativ schlechte mechanische Eigenschaften.

Die DE 100 21 605 A1 offenbart dentale Füllungsmaterialien auf der Basis von Epoxidharzen, die ein Ammoniumsalz, beispielsweise Ammoniumsulfat oder Ammoniumhydrogensulfat, in Kombination mit einem basischen Calciumsalz enthalten. Bevorzugte Calciumsalze sind Calciumoxid und Calciumhydroxid. Bei Zutritt von Feuchtigkeit bildet das Ammoniumsalz durch Reaktion mit dem basischen Calciumsalz Ammoniak, das eine Härtung des Expoxidharzes bewirkt. Außerdem entsteht Gips, der bei der Kristallisation expandiert und so die Randdichtigkeit von Zahn- und Wurzelfüllungen verbessern soll.

Der Erfindung liegt die Aufgabe zugrunde, lagerstabile, selbsthaftende dentale Komposite mit guter Transparenz und guten mechanischen Eigenschaften bereitzustellen, die sich als 2-Komponentensysteme gut mit Doppelschubspritzen mischen und applizieren lassen und die sich insbesondere als dentale Befestigungszemente eignen.

Diese Aufgabe wird durch füllstoffhaltige, radikalisch polymerisierbare Zusammensetzungen gelöst, die mindestens ein saures, radikalisch polymerisierbares Monomer, mindestens einen Fluoroaluminiumsilikatglasfüllstoff und/oder röntgenopaken Glasfüllstoff und mindestens ein bei 20 °C wasserlösliches Sulfat und/oder Phosphat enthalten. Es wurde überraschend gefunden, dass wasserlösliche Sulfate oder Phosphate eine deutliche Erhöhung der Lagerstabilität von Zusammensetzungen bewirken, die saure Monomere in Kombination mit mindestens einem Fluoroaluminiumsilikatglasfüllstoff und/oder röntgenopaken Glasfüllstoff enthalten.

Erfindungsgemäß sind mit Sulfaten vorzugsweise anorganische Salze der Schwefelsäure und mit Phosphaten anorganische Salze der Orthophosphorsäure (H₃PO₄) gemeint. Unter wasserlöslichen Sulfaten bzw. Phosphaten werden vorzugsweise Sulfate bzw. Phosphate mit einer Wasserlöslichkeit von mindestens 100 g/L, vorzugweise von 110 bis 1000 g/L und besonders bevorzugt von 150 bis 800 g/L verstanden.

Bevorzugte wasserlösliche Sulfate sind Kaliumsulfat (K₂SO₄; Wasserlöslichkeit 111 g/L), Natriumsulfat (Na₂SO₄; Wasserlöslichkeit 170 g/L) und besonders bevorzugt Ammoniumsulfat ((NH₄)₂SO₄; Wasserlöslichkeit 754 g/L). Bevorzugte wasserlösliche Phosphate sind Kaliumphosphat (K₃PO₄; Wasserlöslichkeit 508 g/L), Natriumphosphat (Na₃PO₄; Wasserlöslichkeit 285 g/L) und besonders bevorzugt Ammoniumphosphat ((NH₄)₃PO₄; Wasserlöslichkeit 580 g/L). Alle Löslichkeitsangaben beziehen sich auf die Löslichkeit in Wasser bei 20 °C.

Das oder die wasserlöslichen Sulfate und/oder Phosphate werden vorzugsweise in einer Gesamtmenge von mindestens 0,3 Gew.-% und insbesondere mindestens 0,4 Gew.-% zugegeben. Erfindungsgemäß bevorzugt sind Zusammensetzungen, die 0,3 bis 9,0 Gew.-%, besonders bevorzugt 0,4 bis 6,0 Gew.-% und ganz besonders bevorzugt von 0,7 bis 4,0 Gew.-% mindestens eines wasserlöslichen Sulfats und/oder Phosphats enthalten. Alle Prozentangaben hierin beziehen sich, wenn nicht anders angegeben, auf die Gesamtmasse der Zusammensetzung. Die Sulfate oder Phosphate können in gelöster oder vorzugsweise fester Form vorliegen. Sulfate sind erfindungsgemäß bevorzugt.

Die erfindungsgemäßen Zusammensetzungen enthalten mindestens ein radikalisch polymerisierbares Monomer, vorzugsweise ein oder mehrere mono- und/oder polyfunktionelle Monomere. Unter polyfunktionellen Monomeren werden Verbindungen mit zwei oder mehr, vorzugsweise 2 bis 4 und insbesondere 2 radikalisch polymerisierbaren Gruppen verstanden. Monofunktionelle Monomere weisen dementsprechend nur eine radikalisch polymerisierbare Gruppe auf. Polyfunktionelle Monomere haben vernetzende Eigenschaften und werden daher auch als Vernetzermonomere bezeichnet. Bevorzugte radikalisch polymerisierbare Gruppen sind (Meth)acrylat-, (Meth)acrylamid- und Vinylgruppen.

Erfindungsgemäß wird zwischen säuregruppenhaltigen Monomeren und Monomeren unterschieden, die keine Säuregruppen enthalten. Die erfindungsgemäßen Zusammensetzungen enthalten mindestens ein Monomer ohne Säuregruppen und mindestens ein Monomer und/oder Oligomer mit Säuregruppen. Erfindungsgemäß sind Zusammensetzungen bevorzugt, die Monomere mit und Monomere ohne Säuregruppe in einem Gewichtsverhältnis von 1:5 bis 1:36, besonders bevorzugt von 1:6 bis 1:25 und ganz besonders bevorzugt von 1:7 bis 1:20 enthalten.

### Monomere ohne Säuregruppe

Bevorzugt sind Zusammensetzungen, die mindestens ein (Meth)acrylat enthalten, besonders bevorzugt mindestens ein monofunktionelles oder polyfunktionelles Methacrylat, ganz besonders bevorzugt mindestens ein monofunktionelles oder difunktionelles Methacrylat oder eine Mischung davon.

Bevorzugte monofunktionelle (Meth)acrylate sind Benzyl-, Tetrahydrofurfuryl- oder Isobornyl(meth)acrylat, p-Cumylphenoxyethylenglycolmethacrylat (CMP-1E) und 2-([1,1'-Biphenyl]-2-oxy)ethylmethacrylat (MA-836), Tricyclodecanmethyl(meth)-acrylat, 2-(2-Biphenyloxy)ethyl(meth)acrylat. Besonders bevorzugt sind CMP-1E und MA-836.

Gemäß einer Ausführungsform enthalten die erfindungsgemäßen Zusammensetzungen vorzugsweise mindestens ein funktionalisiertes monofunktionelles (Meth)acrylat. Unter funktionalisierten Monomeren werden solche Monomere verstanden, die neben mindestens einer radikalisch polymerisierbaren Gruppe mindestens eine funktionelle Gruppe tragen, vorzugsweise eine Hydroxylgruppe. Bevorzugte funktionalisierte Mono(meth)acrylate sind 2-Hydroxyethyl- und Hydroxyethylpropyl(methacrylat) sowie 2-Acetoxyethylmethacrylat. Besonders bevorzugt ist Hydroxyethylmethacrylat. Die unten genannten säuregruppenhaltigen Monomere sind keine funktionalisierten Monomere im Sinne der Erfindung.

Bevorzugte di- und polyfunktionelle (Meth)acrylate sind Bisphenol-A-dimethacrylat, Bis-GMA (ein Additionsprodukt aus Methacrylsäure und Bisphenol-A-diglycidylether), ethoxy- oder propoxylierte Bisphenol-A-dimethacrylate, wie z.B. das Bisphenol-A-dimethacrylat SR-348c (Sartomer) mit 3 Ethoxygruppen oder 2,2-Bis[4-(2-methacryl-oyloxypropoxy)phenyl]propan, Urethane aus 2-(Hydroxymethyl)acrylsäure und Diisocyanten, wie 2,2,4-Trimethylhexamethylendiisocyanat oder Isophorondiisocyanat, UDMA (ein Additionsprodukt aus 2-Hydroxyethylmethacrylat und 2,2,4-Trimethylhexamethylen-1,6-diisocyanat), Tetramethylxylylendiurethanethylenglykoldi-(meth)acrylat bzw. Tetramethylxylylendiurethan-2-methylethylenglykoldi(meth)acrylat (V380), Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldimethacrylat, 1,10-Decandioldimethacrylat (D₃MA), Bis(methacryl-oyloxymethyl)tricyclo-[5.2.1.0^{2,6}]decan (DCP), Polyethylenglycol- oder Polypropylenglycoldimethacrylate, wie z.B. Polyethylenglycol-200-dimethacrylat oder Polyethylenglycol-400-dimethacrylat (PEG-200- oder PEG-400-DMA) oder 1,12-Dodecandioldimethacrylat. Besonders bevorzugt sind Bis-GMA, UDMA, V-380, Triethylenglycoldimethacrylat (TEGDMA) und PEG-400-DMA (NK-Ester 9G).

Das Monomer Tetramethylxylylendiurethanethylenglykoldi(meth)acrylat bzw. Tetramethylxylylendiurethan-2-methylethylenglykoldiurethandi(meth)acrylat (V380) weist die folgende Formel auf:

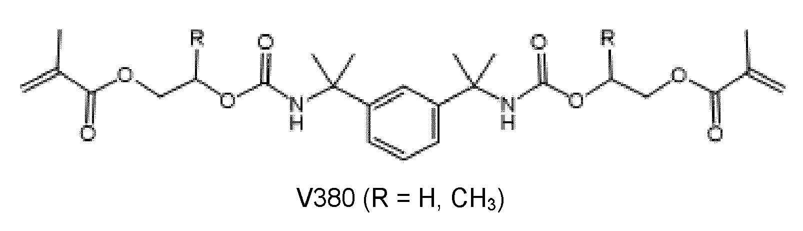

In der gezeigten Formel sind die Reste R unabhängig voneinander jeweils H oder CH₃, wobei die Reste die gleiche Bedeutung oder unterschiedliche Bedeutungen haben können. Vorzugsweise wird eine Mischung eingesetzt, die Moleküle, in denen beide Reste H sind, Moleküle, in denen beide Reste CH₃ sind, und Moleküle enthält, in denen ein Rest H und der andere Rest CH₃ ist, wobei das Verhältnis von H zu CH₃ vorzugsweise 7:3 ist. Eine solche Mischung ist beispielsweise durch Reaktion von 1,3-Bis(1-isocyanato-1-methylethyl)benzol mit 2-Hydroxypropylmethacrylat und 2-Hydroxyethylmethacrylat erhältlich.

Weitere bevorzugte difunktionelle Monomere sind radikalisch polymerisierbare Pyrrolidone, wie z.B. 1,6-Bis(3-vinyl-2-pyrrolidonyl)-hexan, oder kommerziell zugängliche Bisacrylamide wie Methylen- oder Ethylenbisacrylamid, sowie Bis(meth)acrylamide, wie z.B. N,N'-Diethyl-1,3-bis(acrylamido)-propan, 1,3-Bis(methacryl-amido)propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können. Besonders bevorzugt ist N,N'-Diethyl-1,3-bis(acrylamido)-propan (V-392). Diese Monomere zeichnen sich durch eine hohe Hydrolysestabilität aus.

### Säuregruppenhaltige Monomere und Oligomere

Die erfindungsgemäßen Zusammensetzungen enthalten mindestens ein saures, radikalisch polymerisierbares Monomer. Unter sauren, radikalisch polymerisierbaren Monomeren werden Monomere verstanden, die neben mindestens einer radikalisch polymerisierbaren Gruppe mindestens eine Säuregruppe enthalten, vorzugsweise eine Phosphorsäureester-, Phosphonsäure- oder Carboxygruppe, besonders bevorzugt Phosphorsäureester-. Saure Monomere werden hierin auch als Haftkomponente bzw. Haftmonomere oder bezeichnet. Bevorzugt sind Haftmonomere, die als radikalisch polymerisierbare Gruppe mindestens eine (Meth)acrylat- und insbesondere Methacrylatgruppe enthalten.

Bevorzugte säuregruppenhaltige Monomere sind Phosphorsäureester- und Phosphonsäuremonomere. Besonders bevorzugte sind 2-Methacryloyloxyethylphenyl-hydrogenphosphat, 10-Methacryloyloxydecyl-dihydrogenphosphat (MDP) Glycerindimethacrylatdihydrogenphosphat oder Dipentaerythritpentamethacryloyloxyphosphat. 4-Vinylbenzylphosphonsäure, 2-[4-(Dihydroxyphosphoryl)-2-oxa-butyl]-acrylsäure oder hydrolysestabile Ester, wie z.B. 2-[4-(Dihydroxyphosphoryl)-2-oxa-butyl]-acrylsäure-2,4,6-trimethylphenylester. Ganz besonders bevorzugt sind MDP, 2-Methacryloyloxyethylphenyl-hydrogenphosphat und Glycerindimethacrylatdihydrogenphosphat.

Weitere bevorzugte säuregruppenhaltige Monomere sind COOH-gruppenhaltige polymerisationsfähige Monomere. Besonders bevorzugt sind 4-(Meth)acryloyl-oxyethyltrimellitsäureanhydrid, 10-Methacryloyloxydecylmalonsäure, N-(2-Hydroxy-3-methacryloyloxypropyl)-N-phenylglycin und 4-Vinylbenzoesäure.

Gemäß einer weiteren, bevorzugten Ausführungsform enthalten die erfindungsgemäßen Zusammensetzungen zusätzlich mindestens ein saures, radikalisch polymerisierbares Oligomer. Unter Oligomeren werden Polymere mit einem Polymerisationsgrad Pₙ von 2 bis 100 verstanden (Pₙ = Mₙ / Mᵤ; Mₙ: zahlenmittlere Polymermolmasse, Mᵤ: Molmasse der Monomereinheit). Saure, radikalisch polymerisierbare Oligomere weisen mindestens eine Säuregruppe, vorzugsweise Carboxygruppe, und mindestens eine radikalisch polymerisierbare Gruppe, vorzugsweise (Meth)acrylat- und insbesondere Methacrylatgruppe, auf.

Erfindungsgemäß bevorzugte säuregruppenhaltige Oligomere sind oligomere Carbonsäuren, wie z.B. Polyacrylsäure, vorzugsweise mit einer zahlenmittleren Molmasse Mₙ kleiner 7200 g/mol, bevorzugt kleiner 7000 g/mol und besonders bevorzugt kleiner 6800 g/mol, wobei Mₙ bevorzugt in einem Bereich von 800 bis 7200 g/mol, besonders bevorzugt von 500 bis 7000 g/mol und ganz besonders bevorzugt von 500 bis 6800 g/mol liegt. Besonders bevorzugt sind oligomere Carbonsäuren mit (Meth)acrylatgruppen. Diese sind z.B. durch Umsetzung von oligomerer Polyacrylsäure mit Glycidylmethacrylat oder 2-Isocyanatoethylmethacrylat erhältlich.

Wenn nicht anders angegeben, handelt es sich hierin bei der Molmasse von Oligomeren und Polymeren um die zahlenmittlere Molmasse, deren Absolutwerte man mit den bekannten Methoden der Gefrierpunktserniedrigung (Kryoskopie), der Siedepunktserhöhung (Ebullioskopie) oder aus der Erniedrigung des Dampfdrucks (Dampfdruckosmometrie) bestimmen kann. Vorzugsweise wird die zahlenmittlere Molmasse von Oligomeren und Polymeren mittels Gel-Permeations-Chromatographie (GPC) bestimmt. Dabei handelt es sich um eine Relativmethode bei der die Moleküle aufgrund ihrer Größe, genauer gesagt aufgrund ihres hydrodynamischen Volumens, getrennt werden. Die Bestimmung der absoluten Molmasse erfolgt durch Kalibrierung mit bekannten Standards.

Die erfindungsgemäßen Zusammensetzungen enthalten darüber hinaus vorzugsweise auch Wasser. Es wurde gefunden, dass ein Wassergehalt von 1 bis 7 Gew.-%, besonders bevorzugt 1 bis 5 Gew.-%, jeweils bezogen auf die Gesamtmasse der Zusammensetzung, eine Verbesserung der Haftwirkung an Dentin und Zahnschmelz bewirkt.

Die erfindungsgemäßen Zusammensetzungen enthalten außerdem mindestens einen Initiator zur Initiierung der radikalischen Polymerisation, vorzugsweise einen Photoinitiator. Bevorzugte Photoinitiatoren sind Benzophenon, Benzoin sowie deren Derivate, α-Diketone oder deren Derivate, wie 9,10-Phenanthrenchinon, 1-Phenyl-propan-1,2-dion, Diacetyl und 4,4'-Dichlorbenzil. Besonders bevorzugt werden Campherchinon (CC) und 2,2-Dimethoxy-2-phenyl-acetophenon und ganz besonders bevorzugt α-Diketone in Kombination mit Aminen als Reduktionsmittel, wie z.B. 4-(Dimethylamino)-benzoesäureethylester (EDMAB), N,N-Dimethylaminoethylmethacrylat, N,N-Dimethyl-sym.-xylidin oder Triethanolamin verwendet. Weiter bevorzugt sind Norrish-Typ-I-Photoinitiatoren, vor allem Acyl- oder Bisacylphosphinoxide und ganz besonders Monoacyltrialkyl-, Diacyldialkylgermanium- und Tetraacylgermanium-Verbindungen, wie z.B. Benzoyltrimethylgerman, Dibenzoyldiethylgerman, Bis(4-methoxybenzoyl)diethylgerman (Ivocerin^{®}), Tetrabenzoylgerman oder Tetrakis(o-methylbenzoyl)german. Dabei lassen sich auch Mischungen der verschiedenen Photoinitiatoren einsetzen, wie z.B. Bis(4-methoxybenzoyl)diethylgerman oder Tetrakis(o-methylbenzoyl)german in Kombination mit Campherchinon und 4-Dimethylaminobenzoesäureethylester.

Weiter bevorzugt sind Zusammensetzungen, die einen Redoxinitiator zur Initiierung der radikalischen Polymerisation enthalten, vorzugsweise einen Redoxinitiator auf der Basis eines Oxidationsmittels und eines Reduktionsmittels. Bevorzugte Oxidationsmittel sind Peroxide und insbesondere Hydroperoxide. Ein besonders bevorzugtes Peroxid ist Benzoylperoxid. Bevorzugte Hydroperoxide sind die in EP 3 692 976 A1 offenbarten geruchsarmen Cumolhydroperoxid-Derivate, die in EP 21315089.9 offenbarten oligomeren CHP-Derivate und insbesondere 4-(2-Hydroperoxypropan-2-yl)phenylpropionat und Cumolhydroperoxid (CHP).

Bevorzugte Reduktionsmittel zur Kombination mit Peroxiden sind tertiäre Amine, wie N,N-Dimethyl-p-toluidin, N,N-Dihydroxyethyl-p-toluidin, p-Dimethylaminobenzoesäureethylester oder andere aromatische Dialkylamine, Ascorbinsäure, Sulfinsäuren, Thiole und/oder Hydrogensilane.

Bevorzugte Reduktionsmittel zur Kombination mit Hydroperoxiden sind Thioharnstoffderivate, insbesondere die in der EP 1 754 465 A1 in Absatz [0009] aufgeführten Verbindungen. Besonders bevorzugt sind Methyl-, Ethyl-, Allyl-, Butyl-, Hexyl-, Octyl-, Benzyl-, 1,1,3-Trimethyl-, 1,1-Diallyl, 1,3-Diallyl-, 1-(2-Pyridyl-2-thioharnstoff, Acetyl-, Propanoyl-, Butanoyl-, Pentanoyl-, Hexanoyl-, Heptanoyl-, Octanoyl, Nonanoyl, Decanoyl, Benzoylthioharnstoff und Mischungen davon. Ganz besonders bevorzugt sind Acetyl-, Allyl-, Pyridyl- und Phenylthioharnstoff sowie Hexanoylthioharnstoff und Mischungen davon sowie polymerisationsfähige ThioharnstoffDerivate, wie z.B. *N*-(2-Methacryloyloxyethoxysuccinoyl)-thioharnstoff und *N*-(4-Vinylbenzoyl)-thioharnstoff). Außerdem kann vorteilhaft eine Kombination von einem oder mehreren der genannten Thioharnstoffderivate mit einem oder mehreren Imidazolen eingesetzt werden. Bevorzugte Imidazole sind 2-Mercapto-1-methylimidazol oder 2-Mercaptobenzimidazol.

Gemäß einer bevorzugten Ausführungsform können die erfindungsgemäßen Zusammensetzungen neben mindestens einem Hydroperoxid und mindestens einem Thioharnstoffderivat außerdem zusätzlich mindestens eine Übergangsmetallverbindung zur Beschleunigung der Härtung enthalten. Erfindungsgemäß bevorzugte Übergangsmetallverbindungen sind Verbindungen, die sich von Übergangsmetallen ableiten, die mindestens zwei stabile Oxidationsstufen haben. Besonders bevorzugt sind Verbindungen der Elemente Kupfer, Eisen, Kobalt, Nickel und Mangan. Diese Metalle weisen die folgenden stabilen Oxydationsstufen auf: Cu(I)/Cu(II), Fe(II)/Fe(III), Co(II)/Co(III), Ni(II)/Ni(III), Mn(II)/Mn(III). Zusammensetzungen, die mindestens eine Kupferverbindung enthalten, sind besonders bevorzugt. Die Übergansmetallverbindungen werden vorzugsweise in katalytische Mengen eingesetzt, besonders bevorzugt in einer Menge von 10 bis 200 ppm. Diese führen zu keiner Verfärbungen der Dentalmaterialien. Aufgrund der guten Monomerlöslichkeit werden die Übergangsmetalle vorzugsweise in Form ihrer Acetylacetonate, 2-Ethylhexanoate oder THF-Addukte eingesetzt. Bevorzugt sind weiterhin ihre Komplexe mit mehrzähnigen Liganden wie z.B. 2-(2-Aminoethylamino)ethanol, Triethylentetramin, Dimethylglyoxim, 8-Hydroxychinolin, 2,2'-Bipyridin oder 1,10-Phenanthrolin. Ein erfindungsgemäß besonders bevorzugter Initiator ist eine Mischung von Cumolhydroperoxid (CHP) mit mindestens einem der oben genannten Thioharnstoffderivate und Kupfer(II)-acetylacetonat.

Die erfindungsgemäßen Zusammensetzungen enthalten vorzugsweise keine Barbitursäure oder Barbitursäure-Derivate wie 1,3,5-Trimethylbarbitursäure, 1-Benzyl-5-phenylbarbitursäure, 5-Butylbarbitursäure oder 1-Cylohexyl-5-ethylbarbitursäure. Zusammensetzungen, die Barbiturate enthalten, weisen eine unbefriedigende Lagerstabilität auf, weil Barbiturate durch Oxidation mit Luftsauerstoff polymerisationsauslösende Radikale bilden. Außerdem haben Barbiturate nachteilige physiologische Wirkungen wie Bradykardie, Hypotonie oder Blutstörungen.

Die erfindungsgemäßen Zusammensetzungen enthalten mindestens einen anorganischen Füllstoff. Füllstoffhaltige Zusammensetzungen werden als Komposite bezeichnet. Bevorzugt sind Zusammensetzungen, die mindestens einen Fluoroaluminiumsilikatglasfüllstoff (FAS- Füller) und/oder einen röntgenopaken Glasfüllstoff enthalten.

Bevorzugte röntgenopake Glasfüllstoffe weisen die folgende Zusammensetzung auf (Gew.-%): SiO₂: 20-80; B₂O₃: 2-15, BaO oder SrO: 0-40; Al₂O₃: 2-20; CaO und/oder MgO: 0-20; Na₂O, K₂O, Cs₂O: je 0-10; WO₃: 0-20; ZnO: 0-20; La₂O₃: 0-10; ZrO₂: 0-15; P₂O₅: 0-30; Ta₂O₅, Nb₂O₅ oder Yb₂O₃: 0-5; und CaF₂ und/oder SrF₂ 0-10. Besonders bevorzugt sind röntgenopake Glasfüller mit der Zusammensetzung (Gew.-%): SiO₂: 50-75; B₂O₃: 2-15; BaO oder SrO: 2-35; Al₂O₃: 2-15; CaO und/oder MgO: 0-10; und Na₂O: 0-10.

Besonders bevorzugte FAS-Füller haben die folgende Zusammensetzung (Gew.-%): SiO₂: 20-35; Al₂O₃: 15-35; BaO oder SrO: 10-25; CaO: 0-20; ZnO: 0-15; P₂O₅: 5-20; Na₂O, K₂O, Cs₂O: je 0-10; und CaF₂: 0,5-20. Besonders bevorzugt sind FAS-Füller mit der Zusammensetzung (Gew.-%): SiO₂: 20-30; Al₂O₃: 20-30; BaO oder SrO: 10-25; CaO: 5-20; P₂O₅: 5-20; Na₂O: 0-10; und CaF₂: 5-20.

Alle Angaben beziehen sich auf die Gesamtmasse des Glases, wobei alle Komponenten mit Ausnahme von Fluor als Oxide berechnet werden, so wie es bei Gläsern und Glaskeramiken üblich ist.

Die FAS-Füller und röntgenopaken Glasfüller haben vorzugsweise eine mittlere Partikelgröße von 0,2 bis 20 µm und besonders bevorzugt von 0,4 bis 5 µm.

Die erfindungsgemäßen Zusammensetzungen enthalten vorzugsweise 25 Gew.-% bis 80 Gew.-%, besonders bevorzugt 30 bis 75 Gew.-% und ganz besonders bevorzugt 40 bis 70 Gew.-% FAS-Füller und/oder röntgenopaken Glasfüller, jeweils bezogen auf die Gesamtmasse der Zusammensetzung.

Zusätzlich zu den genannten FAS- und röntgenopaken Glasfüllstoffen können die erfindungsgemäßen Zusammensetzungen weitere Füllstoffe enthalten.

Bevorzugte weitere Füllstoffe sind Metalloxide, besonders bevorzugt Mischoxide, die 60 bis 80 Gew.-% SiO₂ und mindestens eines der Metalloxide ZrO₂, Yb₂O₃, ZnO, Ta₂O₅, Nb₂O₅ und/oder La₂O₃ enthalten, so dass sich die Gesamtmenge auf 100 % ergänzt. Mischoxide wie SiO₂-ZrO₂ sind z.B. durch hydrolytische Cokondensation von Metallalkoxiden zugänglich. Die Metalloxide haben vorzugsweise eine mittlere Partikelgröße von 0,05 bis 10 µm, besonders bevorzugt von 0,1 bis 5 µm.

Weitere bevorzugte zusätzliche Füllstoffe sind pyrogene Kieselsäure oder Fällungskieselsäure mit einer Primärpartikelgröße von 0,01-0,15 µm sowie Quarz- oder Glaskeramikpulver mit einer Partikelgröße von 0,1 bis 15 µm, vorzugweise von 0,2 bis 5 µm, sowie Ytterbiumtrifluorid. Das Ytterbiumtrifluorid hat vorzugsweise eine Partikelgröße von 80 bis 900 nm und besonders bevorzugt 100 bis 300 nm. Diese Füller werden vorzugsweise in einer Menge von 0,1 bis 40 Gew.-%, besonders bevorzugt 0,2 bis 35 Gew.-% und ganz besonders bevorzugt in einer Menge von 0,3 bis 25 Gew.-% eingesetzt, jeweils bezogen auf die Gesamtmasse der Zusammensetzung.

Außerdem sind als weitere Füllstoffe sog. Kompositfüller bevorzugt. Diese werden auch als Isofüller bezeichnet. Hierbei handelt es sich um splitterförmige Polymerisate, die ihrerseits einen Füllstoff enthalten, vorzugsweise pyrogenes SiO₂ und/oder Ytterbiumtrifluorid. Bevorzugt sind Polymerisate auf der Basis von Dimethacrylaten. Zur Herstellung der Isofüller werden der oder die Füllstoffe z.B. in eine Dimethacrylatharzmatrix eingearbeitet, die so erhaltene Kompositpaste anschließend thermisch polymerisiert und dann gemahlen.

Ein erfindungsgemäß bevorzugter Kompositfüller lässt sich beispielsweise durch thermische Härtung einer Mischung von Bis-GMA (8,80 Gew.-%), UDMA (6,60 Gew.-%), 1,10-Decandioldimethacrylat (5,93 Gew.-%), Dibenzoylperoxid + 2,6-Di-tert.-butyl-4-methylphenol (zusammen 0,67 Gew.-%), Glasfüller (mittlere Korngröße 0,4 µm; 53,0 Gew.-%) und YbF₃ (25,0 Gew.-%) und anschließendes Vermahlen des gehärteten Materials auf die gewünschte Korngröße herstellen. Alle Prozentangaben beziehen sich auf die Gesamtmasse des Kompositfüllers.

Als weitere Füllstoffe können auch sog. inertisierte Füllstoffe eingesetzt werden. Dabei handelt es sich um Glasfüllstoffe, deren Oberfläche mit einer Diffusionsbarriereschicht, z.B. auf Sol-Gel-Basis, oder mit einer Polymerschicht, z.B. aus PVC, beschichtet ist. Bevorzugt sind die in der EP 2 103 296 A1 beschrieben Füllstoffe.

Zur Verbesserung des Verbundes zwischen Füllstoff und Matrix sind die Füllstoffe vorzugsweise mit methacrylatfunktionalisierten Silanen, wie z.B. 3-Methacryloyloxypropyltrimethoxysilan, oberflächenmodifiziert.

Die erfindungsgemäßen Zusammensetzungen enthalten vorzugsweise 1 bis 50 Gew.-%, bevorzugt 1,5 bis 40 Gew.-% und besonders bevorzugt 2 bis 30 Gew.-% eines oder mehrerer weiterer Füllstoffe, vorzugsweise eines oder mehrerer Metalloxide, pyrogene Kieselsäure und/oder Fällungskieselsäure, jeweils bezogen auf die Gesamtmasse der Zusammensetzung.

Wenn nicht anders angegeben, handelt es sich bei allen Partikelgrößen hierin um volumengemittelte Partikelgrößen (D50-Werte), d.h. 50 % des Gesamtvolumens, das alle Partikel gemeinsam besitzen, ist in Partikeln enthalten, die einen Durchmesser kleiner als den angegebene Wert aufweisen. Der D10-Wert ist dementsprechend der volumetrische Durchmesser bei dem 10% des gesamten Füllstoffvolumens kleiner als der genannte Wert sind.

Die Partikelgrößenbestimmung im Bereich von 0,1 µm bis 1000 µm erfolgt vorzugsweise mittels statischer Lichtstreuung (SLS), beispielsweise mit einem statischen Laserstreuungs-Partikelgrößen-Analysator LA-960 (Horiba, Japan) oder mit einem Microtrac S100 Partikelgrößen-Analysator (Microtrac, USA). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320. Die Messung der Partikelgröße in einem Bereich von 1 nm bis 0,1 µm erfolgt vorzugsweise durch dynamische Lichtstreuung (DLS) von wässrigen Partikeldispersionen, vorzugsweise mit einem He-Ne-Laser mit einer Wellenlänge von 633 nm, bei einem Streuwinkel von 90° und bei 25°C, z.B. mit einem Malvern Zetasizer Nano ZS (Malvern Instruments, Malvern UK).

Im Fall aggregierter und agglomerierter Partikel kann die Primärteilchengröße anhand von TEM-Aufnahmen bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf ein 50 Å dickes Kupfergitter (Maschenweite 300 Mesh) aufgebracht, das mit Kohlenstoff beschichtet ist, und im Anschluss das Lösungsmittel verdampft. Die Partikel werden ausgezählt und der arithmetische Mittelwert berechnet.

Die erfindungsgemäßen Zusammensetzungen können außerdem zusätzlich weitere Additive enthalten, vor allem Stabilisatoren, Farbmittel, Phasentransferkatalysatoren, mikrobizide Wirkstoffe, fluoridionenabgebende Additive, wie z.B. Fluorid-Salze, insbesondere NaF oder Ammoniumfluorid, oder Fluorsilane, optische Aufheller, Weichmacher und/oder UV-Absorber.

Die erfindungsgemäßen Zusammensetzungen enthalten vorzugsweise:
- 5 bis 60 Gew.-%, bevorzugt 8 bis 45 Gew.-% besonders bevorzugt 10 bis 35 Gew.-% mindestens eines radikalisch polymerisierbaren Monomers ohne Säuregruppen,
- 1 bis 15 Gew.-%, bevorzugt 2 bis 12 Gew.-% und besonders bevorzugt 3 bis 10 Gew.-% mindestens eines säuregruppenhaltigen, radikalisch polymerisierbaren Monomers,
- 25 bis 80 Gew.-%, bevorzugt 30 bis 75 Gew.-% und besonders bevorzugt 40 bis 70 Gew.-% mindestens eines FAS-Füllstoffs und/oder röntgenopaken Glasfüllstoffs,
- 0,1 bis 8 Gew.-%, bevorzugt 0,5 bis 6 Gew.-% und besonders bevorzugt 1 bis 5 Gew.-% mindestens eines Initiators für die radikalische Polymerisation und
- 0,3 bis 9 Gew.-%, bevorzugt 0,4 bis 6 Gew.-% und besonders bevorzugt 0,7 bis 4,0 Gew.-% mindestens eines wasserlöslichen Sulfates und/oder Phosphates.

Wenn nicht anders angegeben, beziehen sich alle Prozentangaben hierin auf die Gesamtmasse der Zusammensetzung. Allen Mengenangaben für radikalisch polymerisierbare Monomere (poly- und monofunktionell) beziehen sich nur auf Monomere ohne Säuregruppe und schließen säuregruppenhaltige Monomere nicht ein.

Der Initiator kann ein Redoxinitiator, ein Photoinitiator oder ein Initiator für die duale Härtung sein. Die genannten Mengenangaben enthalten sämtliche Initiatorbestandteile, d.h. die Initiatoren selbst und ggf. Reduktionsmittel, Übergangsmetallverbindung etc. Erfindungsgemäß sind Zusammensetzungen bevorzugt, die mindestens einen Redoxinitiator oder mindestens einen Redoxinitiator und mindestens einen Photoinitiator enthalten.

Erfindungsgemäß sind solche Zusammensetzungen besonders bevorzugt, die die folgenden Bestandteile enthalten:
a) 0,3 bis 9 Gew.-%, bevorzugt 0,4 bis 6 Gew.-% und besonders bevorzugt 0,7 bis 4,0 Gew.-% mindestens eines wasserlöslichen Sulfats und/oder Phosphats,
b) 5 bis 40 Gew.-%, bevorzugt 8 bis 35 Gew.-% besonders bevorzugt 10 bis 30 Gew.-% mindestens eines polyfunktionellen Monomers ohne Säuregruppen,
c) 1 bis 15 Gew.-%, bevorzugt 2 bis 12 Gew.-% und besonders bevorzugt 3 bis 10 Gew.-% mindestens eines säuregruppenhaltigen, radikalisch polymerisierbaren Monomers,
d) 0 bis 10 Gew.-%, bevorzugt 0 bis 8 Gew.-% und besonders bevorzugt 0 bis 5 Gew.-% einer oder mehrerer oligomerer Carbonsäuren,
e) ggf. 0,1 bis 20 Gew.-%, bevorzugt 0,5 bis 15 Gew.-% und besonders bevorzugt 1 bis 10 Gew.-% eines oder mehrerer monofunktioneller Monomere ohne Säuregruppen,
f) 25 bis 80 Gew.-%, bevorzugt 30 bis 75 Gew.-% und besonders bevorzugt 40 bis 70 Gew.-% mindestens eines FAS-Füllstoffs und/oder röntgenopaken Glasfüllstoffs,
g) ggf. 1 bis 50 Gew.-%, bevorzugt 1,5 bis 40 Gew.-% und besonders bevorzugt 2 bis 30 Gew.-% eines oder mehrerer zusätzlicher Füllstoffe,
h) 0,1 bis 8 Gew.-%, bevorzugt 0,5 bis 6 Gew.-% und besonders bevorzugt 1 bis 5 Gew.-% mindestens eines Initiators für die radikalische Polymerisation,
i) 0 bis 20 Gew.-%, bevorzugt 0,2 bis 10 Gew.-% und besonders bevorzugt 1 bis 7 Gew.-% Wasser und
j) 0,01 bis 5 Gew.-%, bevorzugt 0,1 bis 3 Gew.-% und besonders bevorzugt 0,1 bis 2 Gew.-% eines oder mehrerer Additive, jeweils bezogen auf die Gesamtmasse der Zusammensetzung.

Zusammensetzungen, die einen Redoxinitiator enthalten, werden auch als selbsthärtend bezeichnet. Sie werden vorzugsweise in der Form von zwei räumlich getrennten Komponenten, d.h. als 2-Komponenten-System (2K System), eingesetzt. Oxidations- und Reduktionsmittel werden dabei in separate Komponenten der Zusammensetzung eingebarbeitet. Eine Komponente, die sog. Katalysatorpaste, enthält das Oxidationsmittel, vorzugsweise ein Peroxid oder Hydroperoxid, und die zweite Komponente, die sog. Basis-Paste, das entsprechende Reduktionsmittel und ggf. einen Photoinitiator und ggf. katalytische Mengen einer Übergansmetallverbindung. Die Polymerisation wird durch das Mischen der Komponenten gestartet. Zusammensetzungen, die sowohl einen Redox- als auch einen Photoinitiator enthalten, werden als dualhärtend bezeichnet.

Bei zweikomponentigen Zusammensetzungen wird das wasserlösliche Sulfat und/oder Phosphat bevorzugt der Komponente zugesetzt, die das stark saure Haftmonomer, den FAS-Füllstoff und/oder den röntgenopaken Glasfüllstoff enthält.

Erfindungsgemäß sind 2-Komponenten-Systeme bevorzugt. Sie sind vorzugsweise selbsthärtend oder dualhärtend. Die Pasten werden kurz vor der Anwendung vorzugsweise mit einer Doppelschubspritze miteinander gemischt.

Die Katalysatorpaste weist vorzugsweise die folgende Zusammensetzung auf:
a) 0,3 bis 9 Gew.-%, bevorzugt 0,4 bis 6 Gew.-% und besonders bevorzugt 0,7 bis 4,0 Gew.-% mindestens eines wasserlöslichen Sulfats und/oder Phosphates,
b) 5 bis 40 Gew.-%, bevorzugt 8 bis 35 Gew.-%, besonders bevorzugt 10 bis 30 Gew.-% mindestens eines polyfunktionellen Monomers,
c) 2 bis 30 Gew.-%, bevorzugt 4 bis 24 Gew.-% und besonders bevorzugt 6 bis 20 Gew.-% mindestens eines säuregruppenhaltigen Monomers,
d) 0 bis 20 Gew.-%, bevorzugt 0 bis 16 Gew.-% und besonders bevorzugt 0 bis 10 Gew.-% einer oder mehrerer oligomerer Carbonsäuren,
e) ggf. 0,1 bis 20 Gew.-%, bevorzugt 0,5 bis 15 Gew.-% und besonders bevorzugt 1 bis 10 Gew.-% eines oder mehrerer monofunktioneller Monomere,
f) 25 bis 80 Gew.-%, bevorzugt 30 bis 75 Gew.-% und besonders bevorzugt 40 bis 70 Gew.-% mindestens eines FAS- und/oder Glasfüllstoffes,
g) ggf. 1 bis 50 Gew.-%, bevorzugt 1,5 bis 40 Gew.-% und besonders bevorzugt 2 bis 30 Gew.-% eines oder mehrerer zusätzlicher Füllstoffe,
h) 0,01 bis 16 Gew.-%, bevorzugt 0,02 bis 12 Gew.-% und besonders bevorzugt 0,03 bis 10 Gew.-% mindestens eines Peroxids und/oder Hydroperoxids und ggf. mindestens eines Photoinitiators,
i) 0 bis 20 Gew.-%, bevorzugt 0,2 bis 10 Gew.-% und besonders bevorzugt 1 bis 7 Gew.-% Wasser und
j) 0,001 bis 5 Gew.-%, bevorzugt 0,03 bis 3 Gew.-% und besonders bevorzugt 0,05 bis 2 Gew.-% eines oder mehrerer Additive, jeweils bezogen auf die Gesamtmasse der Katalysatorpaste.

Die Basis-Paste weist vorzugsweise folgende Zusammensetzung auf:
a) entfällt,
b) 5 bis 40 Gew.-%, bevorzugt 8 bis 35 Gew.-% besonders bevorzugt 10 bis 30 Gew.-% mindestens eines polyfunktionellen Monomers,
c) entfällt,
d) entfällt,
e) ggf. 0,1 bis 20 Gew.-%, bevorzugt 0,5 bis 15 Gew.-% und besonders bevorzugt 1 bis 10 Gew.-% eines oder mehrerer monofunktioneller Monomere,
f) 25 bis 80 Gew.-%, bevorzugt 30 bis 75 Gew.-% und besonders bevorzugt 40 bis 70 Gew.-% mindestens eines FAS- und/oder Glasfüllstoffs,
g) ggf. 1 bis 50 Gew.-%, bevorzugt 1,5 bis 40 Gew.-% und besonders bevorzugt 2 bis 30 Gew.-% eines oder mehrerer zusätzlicher Füllstoffe,
h) 0,01 bis 16 Gew.-%, bevorzugt 0,02 bis 12 Gew.-% und besonders bevorzugt 0,03 bis 10 Gew.-% mindestens eines geeigneten Reduktionsmittels und ggf. eines Photoinitiators,
i) 0 bis 20 Gew.-%, bevorzugt 0,2 bis 10 Gew.-% und besonders bevorzugt 1 bis 7 Gew.-% Wasser und
j) 0,001 bis 5 Gew.-%, bevorzugt 0,03 bis 3 Gew.-% und besonders bevorzugt 0,05 bis 2 Gew.-% eines oder mehrerer Additive, jeweils bezogen auf die Gesamtmasse der Basis-Paste.

Zur Anwendung werden die Katalysator- und Basis-Paste vorzugsweise in etwa gleichen Anteilen miteinander gemischt. Sie eignen sich daher besonders zur Anwendung mit einer Doppelschubspritze.

Doppelschubspritzen weisen zwei getrennte zylindrische Kammern zur Aufnahme von Basis- und Katalysatorpaste auf. Die Komponenten werden mit zwei miteinander verbundenen Kolben gleichzeitig aus den Kammern herausgedrückt und vorzugsweise durch eine Mischkanüle gepresst und darin miteinander gemischt. Zum Herausdrücken der Pasten kann die Spritze in einen sogenannten Handdispenser eingesetzt werden, der die Handhabung der Spritzen erleichtert.

Die erfindungsgemäßen Zusammensetzungen zeichnen sich durch eine hohe Lagerstabilität und eine verbesserte Transparenz, vorzugsweise größer 10 %, und gute Selbsthaftung auf Schmelz/Dentin aus. Sie eignen sich besonders als Dentalwerkstoffe zur intraoralen Anwendung durch den Zahnarzt zur Restauration geschädigter Zähne (therapeutische Anwendung), insbesondere als dentale Zemente, Beschichtungs- oder Verblendmaterialien, Füllungskomposite und ganz besonders als Befestigungszemente. Die Transparenz wird auf die in den Ausführungsbeispielen beschriebene Weise bestimmt.

Zur Behandlung geschädigter Zähne werden diese vorzugsweise in einem ersten Schritt durch den Zahnarzt präpariert. Anschließend wird mindestens eine erfindungsgemäße Zusammensetzung auf oder in den präparierten Zahn appliziert. Danach kann die Zusammensetzung direkt gehärtet werden, vorzugsweise durch Bestrahlen mit Licht einer geeigneten Wellenlänge, beispielsweise bei der Versorgung von Kavitäten. Alternativ wird eine Dentalrestauration, beispielsweise ein Inlay, Onlay, Veneer, eine Krone, Brücke, ein Gerüst oder eine Dentalkeramik, in den präparierten Zahn eingebracht oder darauf aufgebracht. Die anschließende Härtung der Zusammensetzung erfolgt vorzugsweise durch Licht und/oder durch Selbsthärtung. Die Dentalrestauration wird hierbei am Zahn befestigt.

Die erfindungsgemäßen Zusammensetzungen können auch als extraorale Werkstoffe (nicht therapeutisch) eingesetzt werden, beispielsweise bei der Herstellung oder Reparatur von Dentalrestaurationen. Sie eignen sich zudem als Materialien zur Herstellung und Reparatur von Inlays, Onlays, Kronen oder Brücken.

Zur Herstellung von Dentalrestaurationen wie Inlays, Onlays, Kronen oder Brücken, wird mindestens eine erfindungsgemäße Zusammensetzung auf an sich bekannte Weise zu der gewünschten Dentalrestauration geformt und dann gehärtet. Die Härtung kann durch Licht, Selbsthärtung oder vorzugsweise thermisch erfolgen.

Bei der Reparatur von Dentalrestaurationen werden die erfindungsgemäßen Zusammensetzungen auf die zu reparierende Restauration aufgebacht, beispielsweise um Lücken auszubessern oder um Bruchstücke zu verkleben, und anschließend gehärtet.

Die Erfindung wird im Folgenden anhand von Figuren und Ausführungsbeispielen näher erläutert.

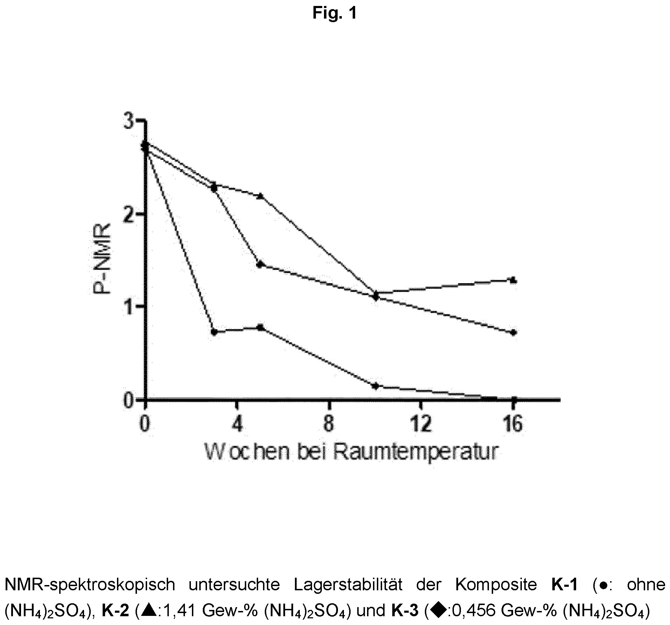

Figur 1 zeigt die Abnahme der Konzentration des sauren Monomers MDP in Abhängigkeit von der Lagerzeit in Kompositpasten mit (- -▲- -; - -◆- -) und ohne (- -■- -) Ammoniumsulfat.

### Beispiel 1

### Untersuchung der Lagerstabilität von selbsthaftenden Kompositen mit und ohne Ammoniumsulfat

Es wurden Kompositpasten K-1 bis K-3 mit den in Tabelle 1 angegebenen Zusammensetzungen (alle Angaben in Gew.-%) aus folgenden Komponenten hergestellt: Glasfüllstoff GM 27884, silanisiert (Schott AG; mittlere Partikelgröße 1 µm; spezifische Oberfläche (BET DIN ISO 9277) 3,9 m²/g; Zusammensetzung (Gew.-%): Al₂O₃: 10, B₂O₃: 10, BaO: 25 und SiO₂: 55), pyrogene Kieselsäure HDK 2000 (Wacker Chemie AG; BET-Oberfläche 120 m²/g), 10-Methacryloyloxydecyldihydrogenphosphat (MDP, Orgentis), Triethylenglycoldimethacrylat (TEGDMA), NK-Ester 9G (Polyethylenglyco-400-dimethacrylat, Kowa Europa GmbH), V-392 (N,N'-Diethyl-1,3-bis(acrylamido)-propan, Ivoclar Vivadent AG), BHT (2,6-Di-*tert*-butyl-*p*-kresol) und Ammoniumsulfat (NH₄)₂SO₄ (Aldrich).

**Tabelle 1: Zusammensetzung der Kompositpasten**

| | **K-1***⁾ **(ohne (NH₄)₂SO₄)** | **K-2** | **K-3** |
|---|---|---|---|
| TEGDMA | 10,96 | 10,62 | 10,66 |
| NK-Ester 9G | 2,34 | 2,36 | 2,37 |
| V-392 | 14,39 | 14,55 | 14,60 |
| MDP | 3,29 | 3,44 | 3,34 |
| BHT | 0,05 | 0,03 | 0,034 |
| (NH₄)₂SO₄ | 0 | 1,41 | 0,456 |
| HDK 2000 | 4,00 | 4,00 | 4,00 |
| GM 27884 | 65,00 | 63,59 | 64,54 |

| | | | |
|---|---|---|---|
| *⁾ Vergleichsbeispiel | | | |

Die Pasten **K-1** (ohne (NH₄)₂SO₄), **K-2** (1,41 % (NH₄)₂SO₄) und **K-3** (0,456 % (NH₄)₂SO₄) wurden für einen Zeitraum von 16 Wochen bei Raumtemperatur gelagert und der Gehalt an MDP wiederholt mittels ³¹P-NMR-Spektroskopie bestimmt. Für die ³¹P-NMR-Messung wurden jeweils 1,5 g der zu analysierenden Probe in ein Zentrifugenröhrchen eingewogen und mit 1,5 ml einer Lösung von 0,1 g Triphenylphosphin in 10 ml deuteriertem Aceton (Aceton-d₆) als innerem Standard versetzt. Die resultierende Suspension wurde mithilfe eines Plattformschüttlers (Vibramax, Heidolph Instruments GmbH & Co. KG, Schwabach) mind. 5 min geschüttelt und anschließend bei 3500 U/min für 15 min zentrifugiert. Der Überstand wurde mittels Einweg-Pipette in ein NMR Probenröhrchen transferiert. Die Messung erfolgte mittels eines Avance DPX 400 (Bruker Spectrospin) 400 MHz - Kernresonanz-Spektrometer. Aus dem Verhältnis der Peakflächen in Bezug auf den inneren Standard wurde die MDP-Konzentration berechnet. Figur 1 zeigt die zeitliche Änderung des Gehalts an MDP in den Kompositpasten mit und ohne (NH₄)₂SO₄.

Die in Fig. 1 dargestellten Ergebnisse belegen eine deutlich verbesserte Lagerstabilität der Komposite **K-2** und **K-3** mit (NH₄)₂SO₄. Das Komposit **K-1** ohne (NH₄)₂SO₄ zeigt eine nahezu vollständige Abnahme des verfügbaren MDP nach nur 10 Wochen, während in den Kompositpasten **K-2** und **K-3** mit (NH₄)₂SO₄ auch nach 16 Wochen noch ein relevanter Gehalt an MDP nachweisbar war.

## Patentansprüche

1. Radikalisch polymerisierbare Zusammensetzung, die mindestens ein saures, radikalisch polymerisierbares Monomer und mindestens einen Fluoroaluminiumsilikatglasfüllstoff und/oder röntgenopaken Glasfüllstoff enthält, **dadurch gekennzeichnet, dass** sie zusätzlich mindestens ein wasserlösliches Sulfat und/oder Phosphat enthält.

2. Zusammensetzung nach Anspruch 1, die als wasserlösliches Sulfat mindestens ein anorganisches Salz der Schwefelsäure mit einer Wasserlöslichkeit von mindestens 100 g/L bei 20 °C, vorzugweise von 110 bis 1000 g/L, und/oder als wasserlösliches Phosphat mindestens ein anorganisches Salz der Orthophosphorsäure mit einer Wasserlöslichkeit von mindestens 100 g/L bei 20 °C, vorzugweise von 110 bis 1000 g/L, enthält.

3. Zusammensetzung nach Anspruch 1 oder 2, die als wasserlösliches Sulfat Kaliumsulfat, Natriumsulfat, Ammoniumsulfat oder eine Mischung davon und/oder als wasserlösliches Phosphat Kaliumphosphat, Natriumphosphat, Ammoniumphosphat oder eine Mischung davon enthält.

4. Zusammensetzung nach einem der Ansprüche 1 bis 3, die 0,3 bis 9,0 Gew.-%, bevorzugt 0,4 bis 6,0 Gew.-% und besonders bevorzugt von 0,7 bis 4,0 Gew.-% mindestens eines wasserlöslichen Sulfats und/oder Phosphats enthält, bezogen auf die Gesamtmasse der Zusammensetzung.

5. Zusammensetzung nach Anspruch 4, die
- 5 bis 60 Gew.-%, bevorzugt 8 bis 45 Gew.-% besonders bevorzugt 10 bis 35 Gew.-% mindestens eines radikalisch polymerisierbaren Monomers ohne Säuregruppen,
- 1 bis 15 Gew.-%, bevorzugt 2 bis 12 Gew.-% und besonders bevorzugt 3 bis 10 Gew.-% mindestens eines säuregruppenhaltigen, radikalisch polymerisierbaren Monomers,
- 25 bis 80 Gew.-%, bevorzugt 30 bis 75 Gew.-% und besonders bevorzugt 40 bis 70 Gew.-% mindestens eines Fluoroaluminiumsilikatglasfüllstoffs - und/oder röntgenopaken Glasfüllstoffs,
- 0,1 bis 8 Gew.-%, bevorzugt 0,5 bis 6 Gew.-% und besonders bevorzugt 1 bis 5 Gew.-% mindestens eines Initiators für die radikalische Polymerisation und
- 0,3 bis 9 Gew.-%, bevorzugt 0,4 bis 6 Gew.-% und besonders bevorzugt 0,7 bis 4,0 Gew.-% mindestens eines wasserlöslichen Sulfates und/oder Phosphats enthält, jeweils bezogen auf die Gesamtmasse der Zusammensetzung.

6. Zusammensetzung nach Anspruch 5, die
a) 0,3 bis 9 Gew.-%, bevorzugt 0,4 bis 6 Gew.-% und besonders bevorzugt 0,7 bis 4,0 Gew.-% mindestens eines wasserlöslichen Sulfats und/oder Phosphats,
b) 5 bis 40 Gew.-%, bevorzugt 8 bis 35 Gew.-% besonders bevorzugt 10 bis 30 Gew.-% mindestens eines polyfunktionellen Monomers ohne Säuregruppen,
c) 1 bis 15 Gew.-%, bevorzugt 2 bis 12 Gew.-% und besonders bevorzugt 3 bis 10 Gew.-% mindestens eines säuregruppenhaltigen, radikalisch polymerisierbaren Monomers,
d) 0 bis 10 Gew.-%, bevorzugt 0 bis 8 Gew.-% und besonders bevorzugt 0 bis 5 Gew.-% einer oder mehrerer oligomerer Carbonsäuren,
e) ggf. 0,1 bis 20 Gew.-%, bevorzugt 0,5 bis 15 Gew.-% und besonders bevorzugt 1 bis 10 Gew.-% eines oder mehrerer monofunktioneller Monomere ohne Säuregruppen,
f) 25 bis 80 Gew.-%, bevorzugt 30 bis 75 Gew.-% und besonders bevorzugt 40 bis 70 Gew.-% mindestens eines Fluoroaluminiumsilikatglasfüllstoffs und/oder röntgenopaken Glasfüllstoffs,
g) ggf. 1 bis 50 Gew.-%, bevorzugt 1,5 bis 40 Gew.-% und besonders bevorzugt 2 bis 30 Gew.-% eines oder mehrerer zusätzlicher Füllstoffe,
h) 0,1 bis 8 Gew.-%, bevorzugt 0,5 bis 6 Gew.-% und besonders bevorzugt 1 bis 5 Gew.-% eines Initiators für die radikalische Polymerisation,
i) 0 bis 20 Gew.-%, bevorzugt 0,2 bis 10 Gew.-% und besonders bevorzugt 1 bis 7 Gew.-% Wasser und
j) 0,01 bis 5 Gew.-%, bevorzugt 0,1 bis 3 Gew.-% und besonders bevorzugt 0,1 bis 2 Gew.-% eines oder mehrerer Additive enthält, jeweils bezogen auf die Gesamtmasse der Zusammensetzung.

7. Zusammensetzung nach einem der vorhergehenden Ansprüche, die als polyfunktionelles Monomer (b) mindestens ein Monomer enthält, das aus Bisphenol-A-dimethacrylat, Bis-GMA (ein Additionsprodukt aus Methacrylsäure und Bisphenol-A-diglycidylether), ethoxy- oder propoxyliertes Bisphenol-A-dimethacrylaten, wie Bisphenol-A-dimethacrylat mit 3 Ethoxygruppen oder 2,2-Bis[4-(2-methacryloyloxypropoxy)phenyl]propan, Urethanen aus 2-(Hydroxymethyl)acrylsäure und Diisocyanten, wie 2,2,4-Trimethylhexamethylendiisocyanat oder Isophorondiisocyanat, UDMA (ein Additionsprodukt aus 2-Hydroxyethylmethacrylat und 2,2,4-Trimethylhexamethylen-1,6-diisocyanat), Tetramethylxylylendiurethanethylenglycoldi(meth)acrylat bzw. Tetramethyl-xylylendiurethan-2-methylethylenglykoldiurethandi(meth)acrylat (V380), Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldimethacrylat, 1,10-Decandioldimethacrylat (D₃MA), Bis(methacryl-oyloxymethyl)tricyclo-[5.2.1.0^{2,6}]decan (DCP), Polyethylenglycol- oder Polypropylenglycoldimethacrylaten, wie Polyethylenglycol-200-dimethacrylat (PEG-200-DMA) oder Polyethylenglycol-400-dimethacrylat (PEG-400-DMA), 1,12-Dodecandioldimethacrylat, radikalisch polymerisierbaren Pyrrolidonen, wie z.B. 1,6-Bis(3-vinyl-2-pyrrolidonyl)-hexan, Bisacrylamiden, wie Methylen- oder Ethylenbisacrylamid, Bis(meth)acrylamiden, wie N,N'-Diethyl-1,3-bis-(acrylamido)-propan, 1,3-Bis(methacrylamido)propan, 1,4-Bis(acrylamido)-butan oder 1,4-Bis(acryloyl)-piperazin, und Mischungen davon ausgewählt ist.

8. Zusammensetzung nach einem der vorhergehenden Ansprüche, die als säuregruppenhaltiges Monomer (c) mindestens ein Monomer enthält, das aus Monomeren, die eine Phosphorsäureestergruppe oder Phosphonsäuregruppe enthalten, vorzugsweise 2-Methacryloyloxyethylphenyl-hydrogenphosphat, 10-Methacryloyloxydecyl-dihydrogenphosphat (MDP) Glycerindimethacrylatdihydrogenphosphat, Dipentaerythritpentamethacryloyloxyphosphat. 4-Vinylbenzylphosphonsäure, 2-[4-(Dihydroxyphosphoryl)-2-oxa-butyl]-acrylsäure und/oder 2-[4-(Dihydroxyphosphoryl)-2-oxa-butyl]-acrylsäure-2,4,6-trimethyl-phenylester, und/oder Monomeren ausgewählt ist, die eine Carboxygruppe enthalten, vorzugsweise 4-(Meth)acryloyloxyethyltrimellitsäureanhydrid, 10-Methacryloyloxydecylmalonsäure, N-(2-Hydroxy-3-methacryloyloxypropyl)-N-phenylglycin und/oder 4-Vinylbenzoesäure.

9. Zusammensetzung nach einem der vorhergehenden Ansprüche, die mindestens eine oligomere Carbonsäure (d) enthält, die aus Polyacrylsäure mit einer zahlenmittleren Molmasse kleiner 7200 g/mol, bevorzugt kleiner 7000 g/mol und besonders bevorzugt kleiner 6800 g/mol ausgewählt ist.

10. Zusammensetzung nach einem der vorangehenden Ansprüche, die mindestens ein monofunktionelles Monomer (e) enthält, das aus Benzyl-, Tetrahydrofurfuryl(meth)acrylat, Isobornyl(meth)acrylat, p-Cumyl-phenoxyethylenglycolmethacrylat (CMP-1E) und 2-([1,1'-Biphenyl]-2-oxy)ethylmethacrylat (MA-836), Tricyclodecanmethyl(meth)acrylat, 2-(2-Biphenyloxy)ethyl(meth)acrylat, 2-Hydroxyethyl(methacrylat), Hydroxyethylpropyl(methacrylat), 2-Acetoxyethylmethacrylat und Mischungen davon ausgewählt ist.

11. Zusammensetzung nach einem der Ansprüche 1 bis 10, die einen röntgenopaken Glasfüllstoff mit der Zusammensetzung (Gew.-%):
SiO₂: 20-80; B₂O₃: 2-15, BaO oder SrO: 0-40; Al₂O₃: 2-20; CaO und/oder MgO: 0-20; Na₂O, K₂O, Cs₂O: je 0-10; WO₃: 0-20; ZnO: 0-20; La₂O₃: 0-10; ZrO₂: 0-15; P₂O₅: 0-30; Ta₂O₅, Nb₂O₅ oder Yb₂O₃: 0-5; und CaF₂ und/oder SrF₂ 0-10; oder vorzugsweise SiO₂: 50-75; B₂O₃: 2-15; BaO oder SrO: 2-35; Al2O₃: 2-15; CaO und/oder MgO: 0-10; und Na₂O: 0-10; und/oder einen Fluoroaluminiumsilikatglasfüllstoff mit der Zusammensetzung (Gew.-%): SiO₂: 20-35; Al₂O₃: 15-35; BaO oder SrO: 10-25; CaO: 0-20; ZnO: 0-15; P₂O₅: 5-20; Na₂O, K₂O, Cs₂O: je 0-10; und CaF₂: 0,5-20 Gew.-%; oder vorzugsweise SiO₂: 20-30; Al₂O₃: 20-30; BaO oder SrO: 10-25; CaO: 5-20; P₂O₅: 5-20; Na₂O: 0-10; und CaF₂: 5-20 Gew.-% enthält, wobei sich alle Angaben auf die Gesamtmasse des Glases beziehen und wobei alle Komponenten mit Ausnahme von Fluor als Oxide berechnet werden.

12. Zusammensetzung nach einem der Ansprüche 6 bis 11, die eine Katalysatorpaste und eine Basis-Paste umfasst, wobei die Katalysatorpaste
a) 0,3 bis 9 Gew.-%, bevorzugt 0,4 bis 6 Gew.-% und besonders bevorzugt 0,7 bis 4,0 Gew.-% mindestens eines wasserlöslichen Sulfats und/oder Phosphats,
b) 5 bis 40 Gew.-%, bevorzugt 8 bis 35 Gew.-%, besonders bevorzugt 10 bis 30 Gew.-% mindestens eines polyfunktionellen Monomers ohne Säuregruppen,
c) 2 bis 30 Gew.-%, bevorzugt 4 bis 24 Gew.-% und besonders bevorzugt 6 bis 20 Gew.-% mindestens eines säuregruppenhaltigen Monomers,
d) 0 bis 20 Gew.-%, bevorzugt 0 bis 16 Gew.-% und besonders bevorzugt 0 bis 10 Gew.-% einer oder mehrerer oligomerer Carbonsäuren,
e) ggf. 0,1 bis 20 Gew.-%, bevorzugt 0,5 bis 15 Gew.-% und besonders bevorzugt 1 bis 10 Gew.-% eines oder mehrerer monofunktioneller Monomere ohne Säuregruppen,
f) 25 bis 80 Gew.-%, bevorzugt 30 bis 75 Gew.-% und besonders bevorzugt 40 bis 70 Gew.-% mindestens eines FAS- und/oder Glasfüllstoffes,
g) ggf. 1 bis 50 Gew.-%, bevorzugt 1,5 bis 40 Gew.-% und besonders bevorzugt 2 bis 30 Gew.-% eines oder mehrerer zusätzlicher Füllstoffe,
h) 0,01 bis 16 Gew.-%, bevorzugt 0,02 bis 12 Gew.-% und besonders bevorzugt 0,03 bis 10 Gew.-% mindestens eines Peroxids und/oder Hydroperoxids und ggf. mindestens eines Photoinitiators,
i) 0 bis 20 Gew.-%, bevorzugt 0,2 bis 10 Gew.-% und besonders bevorzugt 1 bis 7 Gew.-% Wasser und
j) 0,001 bis 5 Gew.-%, bevorzugt 0,03 bis 3 Gew.-% und besonders bevorzugt 0,05 bis 2 Gew.-% eines oder mehrerer Additive enthält, jeweils bezogen auf die Gesamtmasse der Katalysatorpaste,
und wobei die Basis-Paste
a) entfällt,
b) 5 bis 40 Gew.-%, bevorzugt 8 bis 35 Gew.-% besonders bevorzugt 10 bis 30 Gew.-% mindestens eines polyfunktionellen Monomers ohne Säuregruppen,
c) entfällt,
d) entfällt,
e) ggf. 0,1 bis 20 Gew.-%, bevorzugt 0,5 bis 15 Gew.-% und besonders bevorzugt 1 bis 10 Gew.-% eines oder mehrerer monofunktioneller Monomere ohne Säuregruppen,
f) 25 bis 80 Gew.-%, bevorzugt 30 bis 75 Gew.-% und besonders bevorzugt 40 bis 70 Gew.-% mindestens eines FAS- und/oder Glasfüllstoffs,
g) ggf. 1 bis 50 Gew.-%, bevorzugt 1,5 bis 40 Gew.-% und besonders bevorzugt 2 bis 30 Gew.-% eines oder mehrerer zusätzlicher Füllstoffe,
h) 0,01 bis 16 Gew.-%, bevorzugt 0,02 bis 12 Gew.-% und besonders bevorzugt 0,03 bis 10 Gew.-% mindestens eines geeigneten Reduktionsmittels und ggf. eines Photoinitiators,
i) 0 bis 20 Gew.-%, bevorzugt 0,2 bis 10 Gew.-% und besonders bevorzugt 1 bis 7 Gew.-% Wasser und
j) 0,001 bis 5 Gew.-%, bevorzugt 0,03 bis 3 Gew.-% und besonders bevorzugt 0,05 bis 2 Gew.-% eines oder mehrerer Additive enthält, jeweils bezogen auf die Gesamtmasse der Basis-Paste.

13. Zusammensetzung nach Anspruch 12, wobei die Katalysatorpaste und die Basis-Paste jeweils in unterschiedlichen Kammern einer Doppelschubspritze enthalten sind.

14. Zusammensetzung nach einem der Ansprüche 1 bis 13 zur therapeutischen Anwendung als Dentalwerkstoff, vorzugsweise als dentaler Zement, Beschichtungs- oder Verblendmaterial, Füllungskomposit oder Befestigungszement.

15. Nicht-therapeutische Verwendung einer Zusammensetzung gemäß einem der Ansprüche 1 bis 13 zur Herstellung oder Reparatur von Dentalrestaurationen, insbesondere von Inlays, Onlays, Kronen oder Brücken.
